# EUROPEAN PATENT SPECIFICATION

(11) **EP 2 841 447 B1**
(45) Date of publication and mention of the grant of the patent: **13.06.2018**
(21) Application number: 13781268.1
(22) Date of filing: 30.01.2013
(51) Int. Cl.: C07K 1/22, A61M 1/14, A61M 1/34, A61M 1/16, A61K 38/16, C07K 14/47

(54) **NOVEL COMPOSITION FOR EXTRACORPOREAL REDUCTION OF BETA-AMYLOIDS AND PROCESS FOR PRODUCING THEREOF**
NEUARTIGE ZUSAMMENSETZUNG ZUR EXTRAKORPORALEN REDUKTION VON BETA-AMYLOIDEN UND VERFAHREN ZUR HERSTELLUNG DAVON
NOUVELLE COMPOSITION POUR LA RÉDUCTION EXTRACORPORELLE DES BÊTA-AMYLOÏDES ET SON PROCÉDÉ DE PRODUCTION

(30) Priority: 26.04.2012 US 201261638672 P
(43) Date of publication of application: 04.03.2015
(73) Proprietor: Amylex Pharmaceuticals, Inc., Metro Manila 1605 (PH)
(72) Inventor: SANTOS, Rogelio, B., Jr., Metro Manila 1605 (PH); STEIN, Stanley, East Brunswick, New Jersey 08816 (US); KASINATHAN, Chinnaswamy, Holmdel, New Jersey 07733 (US)
(74) Representative: HGF Limited
(86) International application number: PCT/PH2013/000005
(87) International publication number: WO 2013/162387

(56) References cited:
- WO-A1-96/28471
- WO-A2-2007/047967
- JP-A- S4 942 189
- JP-A- H09 157 283
- K KAWAGUCHI ET AL.: "Novel therapeutic approach for Alzheimer's disease by removing amyloid beta protein from the brain with an extracorporeal removal system", JOURNAL OF ARTIFICIAL ORGANS, vol. 13, no. 1, 2010, pages 31-37, XP27440282, SPRINGER VERLAG, TOKYO ISSN: 1434-7229
- SUNDARAM, R.K. ET AL.: 'Detoxification depot for beta-amyloid peptides.' CURRENT ALZHEIMER RESEARCH vol. 5, no. 1, February 2008, pages 26 - 32, XP008175039
- KITAGUCHI, N. ET AL.: 'Reduction of Alzheimer's disease amyloid-beta in plasma by hemodialysis and its relation to cognitive functions.' BLOOD PURIFICATION vol. 32, no. 1, 2011, pages 57 - 62, XP008175041
- RUBIO, I. ET AL.: 'Plasma amyloid-beta, Abetal-42, load is reduced by haemodialysis.' JOURNAL OF ALZHEIMER'S DISEASE vol. 10, no. 4, December 2006, pages 439 - 443, XP008175040

## Description

### FIELD OF THE INVENTION

The present invention generally relates to a composition intended for reducing Beta-Amyloid levels in a subject. More particularly, the present invention relates to use of a composition for the preparation of a dialysis fluid formulation, intended for the method and system of extracorporeal treatment, through a blood filtration process, of a Beta-Amyloid associated pathological condition in the subject.

### BACKGROUND OF THE INVENTION

The hallmark of Alzheimer's disease (AD) is the presence in the brain of senile plaques, which are primarily composed of a central deposition of Beta-Amyloid peptides. Genetic, neuropathological and biochemical evidences have shown that these deposits of Beta-Amyloid peptide play an important role in the pathogenesis of AD. The amyloid cascade hypothesis for AD, as presented by Karran et al, postulates that the deposition of Beta-Amyloid peptide in the brain is the central event in the pathology of AD. This hypothesis has been very influential in research works in the field of both academe and pharmacy, as the same synthesizes histopathological and genetic information. It also provides evidence that the deposition of Beta-Amyloid peptide in the brain parenchyma initiates a sequence of events that ultimately lead to AD dementia. Beta-Amyloid peptide refers to a 39-43 amino acid peptide derived from the amyloid precursor protein (APP) by proteolytic processing as specifically disclosed in U.S. Patent Publication Nos. 2007/0092508 and 2006/0069010, Both Beta-Amyloid 1-40 and Beta-Amyloid 1-42 SEQ ID NOS: 1 and 2, respectively, are components of the deposits of amyloid fibrils found in the brain tissue of AD patients. Beta-Amyloid 1-40 and Beta-Amyloid 1-42 aggregate into insoluble beta-sheet structures which are considered neurotoxic (such as, for example, ADDLs (Amyloid Beta-Derived Diffusible Ligand)). Essentially, ADDLs are soluble oligomers of Beta-Amyloid that accumulate and cause functional deficits prior to overt neuronal cell death or plaque deposition. The process of beta-sheet formation is initiated at Beta-Amyloid residues 16-20 (Beta-Amyloid 16-20 of KLVFF, SEQ ID NOS: 3 and 4) which then nucleates conversion of the entire Beta-Amyloid 1-40 and aggregation of these monomeric Beta-Amyloid peptides into toxic fibrils and plagues has a rate-limiting nucleation phase followed by rapid extension. Indeed, Beta-Amyloid 1-42 is believed to play a more important role in the early nucleation stage.

A Beta-Amyloid peptide sequence comprising residues KLVFF (SEQ ID NO: 4, as mentioned above) is able to bind to the homologous sequence in Beta-Amyloid 1-40 and Beta-Amyloid 1-42 peptides and interferes with fibril formation in vitro and in vivo. KLVFF is found to be the ideal binding site for Beta-Amyloids such that, potentially, there's no organic/natural antibody that could be more efficient than a mechanism that provides targeted capturing and binding action. More specifically, association of the two homologous sequences leads to the formation of an atypical anti-parallel beta-sheet structure stabilize primarily by interaction between Lys, Leu, and Phe residues. This self-recognition property of the KLVFF peptide and the retro-inverso version thereof, FFVLK (SEQ ID NO: 5 of the previously cited U.S. Patent Publications) has been employed in the design of subcutaneous hydrogel "detoxification depots" or "sinks" for the capture of circulating Beta-Amyloid peptides *in vivo.* See, e.g., Zhang et al., Bioconjugate Chem., 2003, 14,86-92; Sundaram et al., c Alzheimer Res., 2008 Feb; 5(1) 26-32; US 2006/0069010; US 2007/0092508.

Although the patterns of symptoms vary among subjects with induced level of neurotoxic Beta-Amyloid peptides, the potential threat of the accumulation of these neurotic peptides which in turn may result in the development of several pathological diseases such as, for example, Alzheimer's Disease (AD) justifies a need for suitable therapy utilizing a composition, as described in the previously cited U.S. Patent Publications, in the great majority of cases. Traditional biopharmaceutical therapy, such as in the form of antibodies administered in a systemic method, although effective during the early phase of treatment, is known to become progressively less effective and efficient if its administration is not consistent throughout a series of treatment schedules. Generally, such anti-bodies come in large molecular sizes. Unlike substances with low molecular weight which can be administered in many different ways depending upon the condition of patient and method of treatment required, the large-sized molecules of most anti-bodies have a limited route for administration. Although anti-bodies are specific to substrates, they are not necessarily specific to targeted site because of the aforementioned limited route for administration. In one instance, it has been known in the art that engineered anti-bodies are too large to cross the blood brain barrier which separates the circulating blood from the brain extracellular fluid (BECF) in the central nervous system, serving as physiological defense mechanism. Among other notable disadvantages of using anti-bodies are their tendencies to provide undesired biological response, cross-reactions with unrelated antigens, and economic burden of patients who have to make commitments with expensive therapeutic procedures over a long period. In view of these disadvantages that are apparent in the art for utilizing anti-bodies, a need for extracorporeal treatment for reducing Beta-Amyloid levels is strongly desired.

Nobuya and Kazunori (2012), through their U.S. Patent Publication No. 2012/0031840 which was filed on 08 July 2012, discloses a method for reducing a Beta-Amyloid concentration in blood, comprising the steps of; removing blood out of a body, passing the blood that is removed through a hollow fiber membrane, and returning the blood that is passed through into the body, wherein the blood containing a β-amyloid-albumin complex is passed through the hollow fiber membrane to allow beta-amyloid to adsorb to the hollow fiber membrane so that the beta-amyloid concentration in blood is reduced. Nobuya and Kazunori do not disclose a composition having a capturing and binding agent which mainly consists of KLVFF peptide sequence or any variant thereof. In their disclosure, it is apparent that the capturing agent used is a polymer selected from the group not consisting of KLVFF or any variant thereof. It is worth noting that the absorbent (itself serving as a Beta-Amyloid binding agent), as disclosed by Nobuya and Kazunori, is introduced in the hollow fiber membrane. After which, the blood is passed through the hollow fiber membrane to remove Beta-Amyloids, and the blood is ultimately returned back to the body. Generally, Nobuya and Kazunori disclose a process that is laborious and hard to maintain. As one having ordinary skill in the art would know, the degree to which a particular binding action occurs in the membrane depends on a large extent and significant considerations. As a result, a substantial amount of effort has to be exerted to study and focus on the characteristics of the membrane. The characteristics of the dialysis membrane, whether it is porous or non-porous, hydrophobic or hydrophilic, polar or non-polar, have to be studied carefully in order to achieve an optimum filtering capacity.

Moreover, Bias et al (2007), through their U.S. Patent Publication No. 20070010435 which was filed on 18 December 2003, teach a method of treating an amyloid disease in a patient in need of such treatment comprising filtering the blood of the patient through a filter, membrane or column, thereby removing circulating beta-amyloid from the patient. Bias et al teach a method that behaves substantially similar to the process described by Nobuya and Kazunori. The similarities lie on the fact that both of the two prior art documents utilize respective Beta-Amyloid binding agents that are introduced into the membrane portion of a dialysis machine. Bias et al only claims a compound for the binding agent that is selected from apolipoprotein E, apolipoprotein J, serum amyloid P component, a RNA aptamer directed against beta-amyloid, α1-antichymotrypsin, a proteoglycan, a ganglioside, vimentin, vitronectin, albumin, transthyretin, amyloid-beta-binding fragments thereof, and combinations thereof. Apparently, the use of KLVFF as capturing and binding agent is not disclosed by Blas et al. In view of the disclosure from Nobuya and Kazunori and from Blas et al, there is still a need for a process that is configured to remove Beta-Amyloids without having to rely mainly on the performance of the semi-permeable membrane or, more specifically, columns. As a matter of fact, Santoro and Guadagni (2009), in their publication entitled, "Dialysis Membrane: from Convection to Adsorption," specify that membrane performance is difficult to evaluate, and different membranes can only be compared by establishing adequate points of comparison. They further elaborate on the count that the points of comparison themselves may change depending on the type of co-morbidities of the specific subject or patient who is considered for membrane selection.

Finally, Kitaguchi et al (2011), in their journal which was published on 24 February 2011 and entitled "Reduction of Alzheimer's Disease Amytoid-β in Plasma by Hemodialysis and Its Relation to Cognitive Functions," reveal that dialyzers, being a primary component of a hemodialysis process, effectively reduce Beta-Amyloids in whole body circulation, and that repeated rapid decrease of plasma Beta-Amyloids may maintain cognitive state. With more particularity, Kitaguchi et al, in the discussion portion of their published journal, identify at least three (3) possible mechanisms to achieve fairly high removal efficacies of dialyzers, namely, filtration, adsorption, or filtration and adsorption (a combination). The authors also emphasize that their preliminary in vitro experiments indicate that the adsorption may have a major contribution in Beta-Amyloid removal activity of the dialyzer. Plasma filtration requires dialysate formulated at certain conductivity value based on input variables such as plasma ultrafiltrate conductivity for feeding on a computer program that is developed to generate conductivity kinetic model. It bears stressing that Kitaguchi et al do not provide description for the process associated with the aforementioned filtration and adsorption. Nor do they disclose an amyloid capturing and binding process associated with any amyloid binding agent, if there is indeed any. Thus, a related disclosure by Nalesso and Ronco (2006) is herein incorporated and quoted. Nalesso and Ronco, in the book edited by Jean-Louis Vincent and entitled "Intensive Care Medicine: Annual Update 2006," describe that plasma filtration and adsorption are two processes which may be coupled together to improve the effectiveness of extracorporeal purification. They further describe that such plasma filtration and adsorption is directed to the utilization of vehicle of pathological molecules. As such, molecules in the whole blood circulation are transported by plasma water if they are soluble or by carriers in the plasma (e.g., albumin or other specific carriers) if they are insoluble. Therefore, the foregoing observation indicates that the real vehicle of toxic molecules is none other than plasma, and the best extracorporeal purification technique could act directly on this plasma. By identifying the carriers and solute composition of the plasma, the plasma itself can become a medium of blood purification. This concept matured into the development of the plasma filtration adsorption dialysis (PFAD). It is believed that Kitaguchi et al have adapted the concept that the plasma is the only carrier of all molecules, including those that are considered toxic. As disclosed by Nalesso and Ronco, all molecules are transported either in the plasma water or bound to albumin (or other protein carrier for that matter). Albumin and other carriers are often present in the solution in plasma water. Thus, transport of molecules depends on their hydrophobic characteristics and their molecular weights. Molecules with elevated solubility are transported in solution found in plasma water; whereas, hydrophobic molecules are transported bound to specific or non-specific carriers in plasma water. Hence, physio-chemical equilibrium can be achieved among tissues and plasma through the capillary endothelium. This results in the establishment of the dynamic equilibrium in the plasma. Consequently, an extracorporeal treatment acting directly on plasma is able to remove toxic molecules from tissues operating on this dynamic equilibrium. As a summary, the process described by Kitaguchi et al involves removal of Beta-Amyloid through the use of ultra-filtrated water to instigate capturing of Beta-Amyloid having low molecular weight. Hence, the described process does not, in any way, involve targeting of specific Beta-Amyloids.

By further analyzing the foregoing concept as explained in full detail by Nalesso and Ronco, a person having ordinary skill in the art can understand the potential of a subject's plasma to be used as dialysate after purification into its component such as electrolyte composition, acid-base status, water, and protein carrier. According to the authors, plasma does not contain cells so they can be used in extracorporeal purification systems using non-biocompatible materials such as some sorbents for specific molecules. To summarize, the patient's own plasma plays a very important function in purifying blood and, consequently, in removing all molecules with high molecular weight or those with hydrophobic characteristics. Kitaguchi et al specify that hydrophobicity may be a key factor of their disclosed dialysis process. Indeed, the plasma is the medium through which all molecules can be transported to the site of purification. Due to the inherent capacity of plasma to bind and transport toxins, the regenerated plasma from the patient can be used. Through the use of regenerated, filtered plasma in a suitable compartment of a dialysis machine, the dialysis procedure based on the processes of diffusion and binding to remove certain toxins may be performed. However, regeneration of plasma may take a longer while. Most notably, one of the major disadvantages that can be observed in plasma regeneration is the potential loss of vital physiological substances from the plasma, and, in most cases, these substances have to be infused back into the patient. This infusion without a doubt provides a more tedious, complicated and expensive process, not to mention that the health risk of the patient is also at stake. Through this process, there is also no guarantee that the escape of trapped Beta-Amyloids back into the blood plasma can be prevented.

In view of all the foregoing limitations of the prior art, it is clear that there is still a need for a compact, inexpensive, and simple standard dialysis machine-assisted process that extracorporeally removes Beta-Amyloids without allowing escape of the Beta-Amyloids back into a subject's body, without having to intricately evaluate the performance and characteristics of a dialysis membrane, and without putting the health condition of the subjects at risk.

### SUMMARY OF THE INVENTION

In view of the foregoing limitations of the prior art, the present invention primarily provides use of a composition of claim 1 that can be utilized for treating a subject with pathological condition related to induced levels of Beta-Amyloids in which this composition is used as part of a dialysis process, characterized in that the composition specifically targets and binds to Beta-Amyloids, that the binding reaction to Beta-Amyloids of the composition occurs extracorporeally, that the composition does not allow escape of captured Beta-Amyloids, and that the composition is mixed with a dialysate for use in dialysis process and, by means of which, the dialysis process results in highly reduced Beta-Amyloid levels in the subject.

It is therefore the primary object of the present invention to provide a composition which can be used to prepare a dialysis fluid or dialysate formulation intended for extracorporeally treating, assisted by a blood filtration process, a pathological condition associated with induced level of Beta-Amyloids. In accordance with the present invention, the composition mainly consists of a binding agent which is designed and prepared to capture and bind target neurotoxic Beta-Amyloids. The capturing agent, also serving as binding agent, is directly introduced into the dialysis fluid or dialysate together with other standard components of an ordinary dialysate required to achieve the equilibrium necessary for the dialysis procedure to take place.

It is another object of the present invention to prepare a composition to treat a subject having a pathological condition related to induced level of Beta-Amyloids and one or more of the symptoms of such pathological condition as mentioned above by providing a composition comprising the KLVFF peptide, or any variant thereof such as its reverse analog FFVLK peptide, in an amount sufficient to capture the majority of prevalent neurotoxic Beta-Amyloids.

It is yet another object of the present invention to provide a capturing and binding agent that includes peptides consisting of an amino acid sequence selected from the group consisting of: Lys-Leu-Val-Phe-Phe-Cys; phe-phe-val-leu-lys-cys; [phe-phe-val-leu-lys-βAla]₂-lys-cys; [phe-phe-val-leu-lys-PEG-lys-]₃-cys; and [phe-phe-val-leu-lys-PEG-lys-]3-cys. An amino acid sequence may include D-isomers which may be prepared as a linear, branched, or cross-linked polypeptide. KLVFF-related peptides could be monomers, dimers, trimers or higher oligomers linked to one another in a linear or branched form, such as, but not limited to the following table:

| Structure of conjugate | Copies of peptide |
|---|---|
| Lys-Leu-Val-Phe-Phe-Cys | 1 (native) |
| phe-phe-val-leu-lys-cys | 1 (retro-inverso) |
| [phe-phe-val-leu-lys- βAla]₂-lys-cys (branched) | 2 (retro-inverso) |
| [phe-phe-val-leu-lys-βAla]₄-lys₂-lys-cys (branched) | 4 (retro-inverso) |
| [phe-phe-val-leu-lys-PEG-lys-]₃-cys (linear) | 3 (retro-inverso) |

Lower case is for D-amino acids. βAla is beta-alanine, C-terminus is amidated, uncharged form, N-terminus is free, positive charged form, PEG can be terminated by an amino group at one end and a carboxylate group at the other end. In a preferred embodiment, the cysteine residue is linked via its side chain thiol to the gel matrix.

It is yet another object of the present invention to use the above-mentioned composition in preparing a dialysis fluid or dialysate intended for further use in a standard or typical dialysis device. This blood filtration device is configured to separate plasma constituents from other cellular components of the blood. The blood filtration device (or a dialyzer) includes three (3) primary components, namely, a blood circuit side adapted to receive extracted blood from a subject, a dialysate side adapted to receive the dialysis fluid formulation, and a permeable membrane that separates the blood circuit side and the dialysate side. In a straightforward sense, the composition has to be introduced in the dialysate contained in the dialysate side of the blood filtration device so the entire object of the invention can be achieved.

It is a further object of the present invention to provide a process of producing a composition, wherein at least one capturing and binding agent, as described above, or one of its pharmaceutically acceptable derivative or analogue, is brought into dosage form together with at least one solid, liquid, or semi-liquid carrier and/or auxiliary substance. With regard to this specific aspect of the present invention, the carrier includes polyethylene glycol polymer chains that can be cross-linked to increase the total molecular weight of the composition. The carrier provides a framework by which the avidity of binding can be increased by linking together multiple copies of the binding element.

It is a further object of the present invention to provide a system for an extracorporeal system for reducing the level of targeted Beta-Amyloid peptides in blood. The system primarily comprises a blood filtration device and a composition which consists mainly of a capturing and binding agent for capturing and binding targeted Beta-Amyloid peptide. In a preferred embodiment of the present invention, the blood filtration device is composed of a blood circuit side, a dialysate side, and a semi-permeable membrane configured to separate plasma constituents from other cellular components of the blood. In another preferred embodiment, the composition is introduced directly into the dialysate contained in the dialysate side.

It is a further object of the present invention to provide a composition for use in a method for reducing Beta-Amyloid levels in the blood of a subject requiring such treatment by extracorporeal circulation. The method primarily comprises of at least four (4) steps. The second step is extracting blood from the subject at a pre-determined flow rate through a blood filtration device having mainly a dialysate side and a blood circuit side wherein the blood is routed to the blood circuit side only. The first step is introducing an effective amount of a composition consisting mainly of a capturing and binding agent for Beta-Amyloid peptides directly into the dialysis fluid contained in the dialysate side. The third step is circulating the blood through the blood circuit side that is located along the periphery of the dialysate side, wherein the circulation is facilitated by any standard dialysis process or, more specifically, hemodialysis process. Finally, the fourth step is returning the other cellular components and treated plasma constituents of the blood back to the subject.

It is a further object of the present invention to provide a package for treatment of patients suffering pathological conditions associated with induced level of Beta-Amyloids. The kit may comprise a quantity of a carrier carrying the Beta-Amyloid capturing and binding agent, in combination with a dialysate material for use in a dialysis procedure. In one embodiment, the composition is combined with sodium bicarbonate in the form of either powder or liquid. In another embodiment, the composition is combined with dialysate acid mixture. In another embodiment, the composition is introduced directly to the dialysate.

Yet another object of the present invention is treating a subject suffering from a pathological condition selected from a group consisting of: Alzheimer's disease (AD), diabetes, Parkinson's Disease, Huntington's Disease, cataracts, muscular dystrophy and Down's Syndrome.

Further objects and accompanying advantages afforded by the present invention will become apparent from the detailed descriptions of the embodiments, as set forth hereunder; to wit:

### BRIEF DESCRIPTION OF THE DRAWINGS

The appended drawings are presented to further describe the present invention and to provide a clear understanding of various embodiments of the present invention through illustrative examples. These examples, being said as illustrative, should not be regarded as limiting with respect to the scope of the present invention.
FIG. 1 shows an illustration of a partial sequence of APP770 wherein KLVFF, primarily serving as capturing and binding agent for mixing with a dialysate solution, is underlined;
FIG. 1A shows a tetramer peptide of the capturing and binding agent as described in FIG. 1;
FIG. 1B shows an illustration of the tetramer peptide of the capturing and binding agent linked to an 8-arm polyethylene glycol maleimide;
FIG. 2 shows a block diagram illustrating the use of a capturing and binding agent, such as that illustrated in FIGS. 1A and 1B, in the preparation of a dialysis fluid formulation for use in a dialysis process;
FIG. 3 shows a block diagram illustrating an extracorporeal system for reducing the level of targeted Beta-Amyloid peptides in blood; and
FIG. 4 shows a flowchart illustrating method of reducing Beta-Amyloid level in the blood of a subject requiring such treatment by extracorporeal blood circulation.

### DETAILED DESCRIPTION OF THE INVENTION

The present invention primarily provides a use of a composition for the preparation of a dialysis fluid formulation intended for the extracorporeal treatment, through a blood filtration process, of a pathological condition associated with the induction of Beta-Amyloid level in a subject. The composition advantageously consists of a capturing and binding agent for capturing and binding targeted Beta-Amyloids, the use of which has been described in the cited prior art documents to be clinically safe and effective in reducing Beta-Amyloid levels in blood of subjects that are suffering from pathological conditions related to induced level of Beta-Amyloids and one or more of the clusters of symptoms that are characteristically exhibiting high level of beta amyloids.

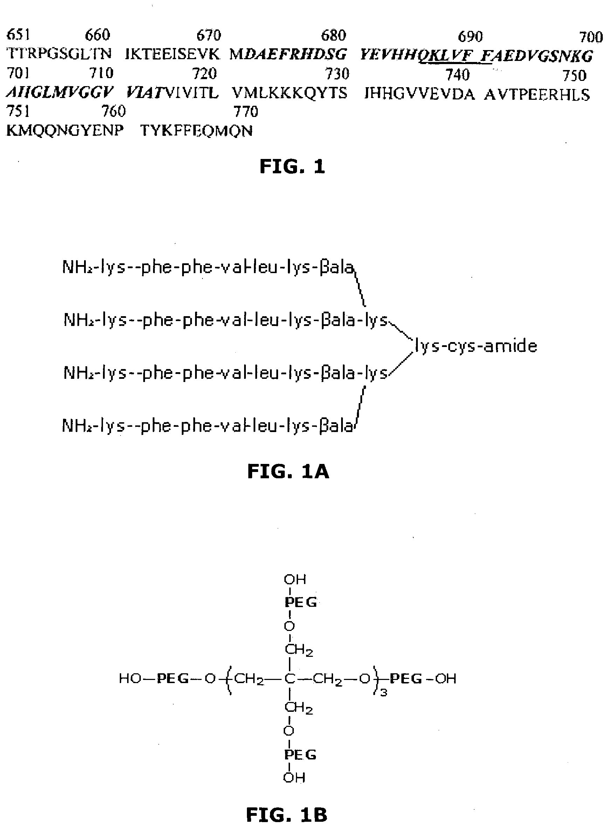

Referring to FIG. 1, there is shown an illustration of partial sequence of APP770. The β-amyloid peptide, Aβ1-42, (SEQ ID NO: 1) is shown in bold italics. On the other hand, Aβ1-40 (SEQ ID NO: 2) would have IAT truncated from the C-terminus. Lastly, KLVFF (SEQ ID NO. 4) is underlined. The KLVFF peptide, or any of its variants, is the primary component of the claimed composition of the present invention. This composition would be superior to attract, capture, and bind neurotoxic Beta-Amyloid peptides that are often present in blood or, more specifically, in the plasma component of the blood. The characteristics of KLVFF peptide and its reverse analog form, FFVLK peptides, have been described by Zhang et al in their prior publications.

The KLVFF-related peptides could be monomers, dimers, trimers or higher oligomers linked to one another in a linear or branched form, such as, but not limited to the following Table:

**Table 1.**

| **Structure of conjugate** | **Copies of peptide** |
|---|---|
| Lys-Leu-Val-Phe-Phe-Cys | 1 (native) |
| phe-phe-val-leu-lys-cys | 1 (retro-inverso) |
| [phe-phe-val-leu-lys-βAla]2-lys-cys (branched) | 2 (retro-inverso) 4 (retro-inverso) |
| [phe-phe-val-leu-lys-PEG-lys-]3-cys (linear) | 3 (retro-inverso) |

Lower case is for D-amino acids. βAla is beta-alanine, C-terminus is amidated, uncharged form, N-terminus is free, positive charged form, PEG can be terminated by an amino group at one end and a carboxylate group at the other end. In a preferred embodiment, the cysteine residue is linked via its side chain thiol to the carrier.

Referring to FIG. 1A, there is shown the composition of the capturing and binding agent having a tetramer peptide containing four copies of the monomer peptide in reverse sequence (retro-inverso), ffvlk, as shown below:

NH2-lys--phe-phe-val-leu-lys-βala
NH2-lys--phe-phe-val-leu-lys-βala-lys
lys-cys-amide
NH2-lys--phe-phe-val-leu-lys-βala-lys
NH2-lys--phe-phe-val-leu-lys-βala

The above structure is preferably provided in a bicarbonate powder which may be mixed with a standard dialysis fluid. A standard dialysis fluid is further preferred to take the form of ultrapure dialysate. An ultrapure type of dialysate is a combination of water and other chemicals that wash waste out of the blood. The primary advantage of using an ultrapure dialysis fluid is that there would be less risk of blood pressure drops during the process of treatment. This ensures that the circulation process is taking place consistently, and there is a stable environment for the capturing and binding agent, KLVFF or FFVLK peptides, to capture and bind the Beta-Amyloids which may be potentially present in the plasma component of the blood.

Referring to FIG. 1B, depicted is a the tetramer capturing and binding agent that is linked, through its *cys* side chain, to an 8-arm polyethylene glycol maleimide to form a molecule of the capturing and binding agent with 32 Beta-Amyloid capturing arms, as shown below:
8ARM-PEG-OH: 8-arm Polyethylene Glycol (tripentaerythritol core) MW: 40,000 Da

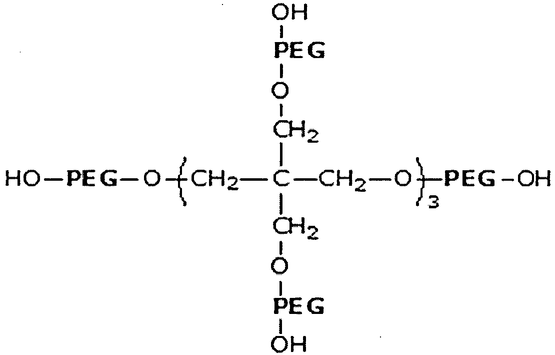

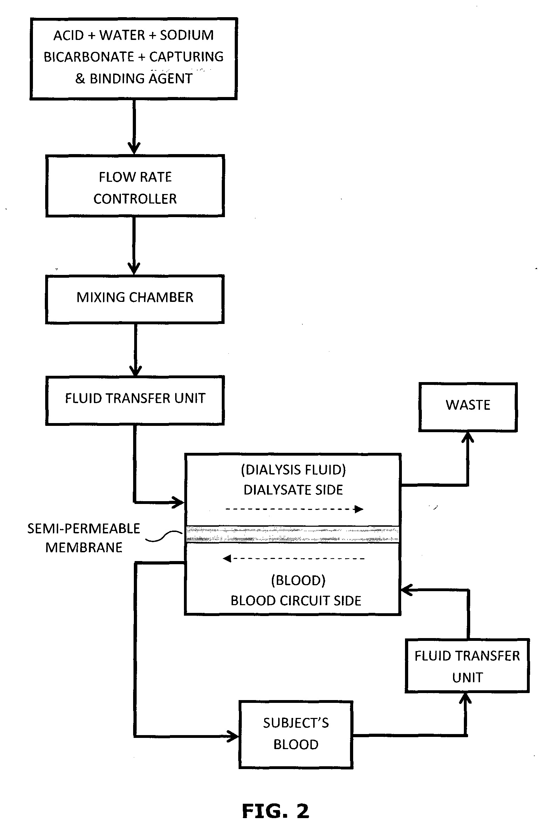

Referring now to FIG. 2, there is shown a block diagram illustrating the use of a binding agent, such as that illustrated in FIG. 1A and 1B, in the preparation of a dialysis fluid formulation. The composition, which mainly consists of the binding agent as described in FIG. 1, may further consist of an effective amount of each of the following components: acid, water, and sodium bicarbonate. The resulting composition may then be suitably mixed with a standard dialysate solution in a mixing chamber. In order to ensure that a suitable amount of each of the resulting composition and the dialysate concentrate solution, individual flow rate controllers may then be used. The resulting mixture now forms a dialysis fluid which may be transferred from the mixing chamber to the dialysate side through a fluid transfer unit with sufficient power to pump an amount of dialysis fluid required in a preferred mode of operation of the present invention. Adjacent the dialysate side is the blood circuit side. The blood circuit side is configured to receive blood from another fluid transfer unit which is designed to pump the blood from a subject at a pre-determined flow rate. In between the dialysate side and the blood circuit side is the permeable membrane. This membrane is characterized by a porous material having pores of sufficient size to permit neurotoxic Beta-Amyloid peptides to pass through it but of certain size so as not to allow the passage of other cellular components of the blood, most of which are generally important as the same is required in maintaining the physiological state thereof. It is the attracting and binding capacity of the capturing and binding agent that plays an important role in the process of substantially eliminating any amount of neurotoxic Beta-Amyloid peptides that may be present in the blood freshly extracted from a patient. While the blood containing neurotoxic Beta-Amyloids is passed through the blood circuit side in a first uniform direction, the capturing and binding agent, being used in the preparation of the dialysis fluid flowing inside the dialysate side in a uniform direction opposite to the first direction, is capturing substantially most of the Beta-Amyloids therein. The capturing and binding agent attracts the Beta-Amyloids which pass through the permeable membrane. The capturing and binding agent itself may be formulated to become large enough so it may not pass through the porous material of the permeable membrane. In this regard, only the Beta-Amyloids having sizes (or molecular weights) that are smaller than the porous material of the permeable membrane can pass through it.

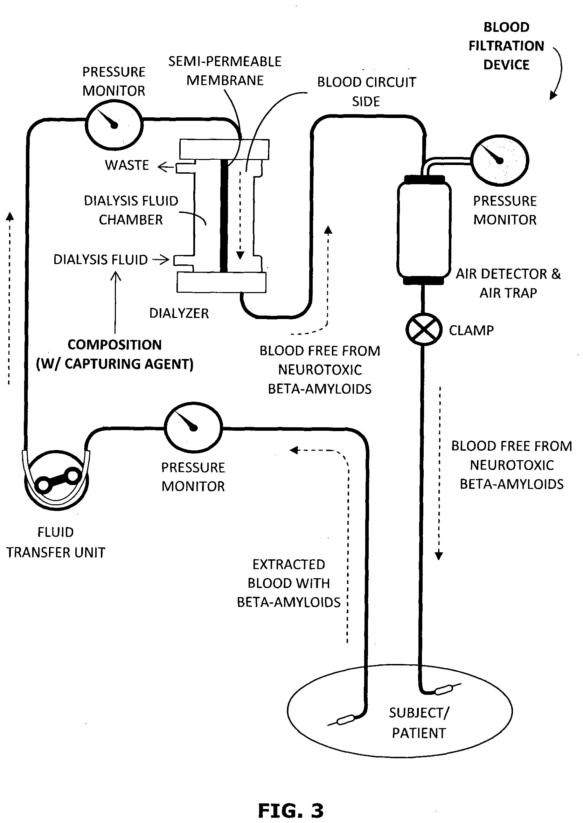

Referring now to FIG. 3, there is shown a block diagram illustrating an extracorporeal system for reducing the level of targeted Beta-Amyloid peptides in blood. The system primarily comprises of two components, namely, a blood filtration device and the composition mainly consisting of a capturing and binding agent, as described in FIG. 1A & 1B. The blood filtration device is configured to separate plasma constituents from other cellular components of the blood. Furthermore, the blood filtration device includes a blood circuit side adapted to receive the extracted blood, a dialysate side adapted to receive a dialysis fluid, and a permeable membrane that separates the blood circuit side and the dialysate side. On the other hand, the second component, being the composition as previously mentioned and described, consists mainly of a binding agent for targeted Beta-Amyloid peptides. The composition is introduced directly into the dialysis fluid contained in the dialysate side. The binding agent has a binding capacity sufficient to capture and bind the neurotoxic Beta-Amyloid peptides from the plasma constituent transfer from the blood circuit side to the dialysate side. In a preferred embodiment, the permeable membrane has pore sizes that are substantially larger than the sizes of the Beta-Amyloid peptides (in oligomer form) thereby enabling the Beta-Amyloid peptides to pass through the permeable membrane while the binding agent attracts them for disposal. In the entire blood filtration process, auxiliary components may be added in the system to ensure reliability in operation. Some of the known auxiliary components which are known in the art are pressure monitor equipment and fluid transfer units which are capable or pumping blood. In another preferred embodiment, air detector and air trap, including clamps, may also be employed in the system so that, substantially, no amount of air can get through the blood. As one having ordinary skill in the art, even a very small amount of air that penetrates into a blood circulation may cause air embolism or gas embolism. This is a pathological condition that is primarily caused by the presence of gas bubbles in a vascular system. Through the use of air trap, the vascular system can still function desirably. Furthermore, a fluid volume controller (not shown) may also be employed in the system. This type of controller is commonly used to achieve fluid balance which, in turn, affects the efficiency of the entire system of providing the dialysis process, as preferably described above.

Generally speaking, dialysis machines are used in the treatment of various diseases, such as kidney related diseases. When the kidneys are functioning normally, they participate in the removal of toxic substances from the body. However, when the kidneys are not functioning properly because of diseases, it is often necessary to remove toxins from the body by a technique known as dialysis. The patient is connected to a dialysis machine so that either blood or peritoneal fluid can flow from the patent, into the machine, and then be returned back to the patient. During this standard process of dialysis, the fluid comes in contact with a dialysis membrane. The dialysis membrane is porous and allows low molecular weight substances, including toxins, to pass through. Such machines, and the use of such machines, are familiar to one having ordinary skill in the art.

In one embodiment of the present invention, a dialysis machine similar to that used in the removal of toxic substances from a patient with kidney disease is used. Using a typical dialysis machine, the inventive technique comprises the incorporation of a "capturing and binding agent" into the dialysis buffer solution which is then used in conjunction with a dialysis machine for treatment of Alzheimer's Disease (AD) by Beta-Amyloid extraction therapy as contemplated herein and, for example, according to the basic scientific principles described in the cited prior art documents. In another preferred embodiment, this capturing and binding agent is the KLVFF peptide.

As familiar to one of ordinary skill in the art, there are two flow paths in a typical dialysis machine. One path provides a circuitous flow of blood or peritoneal fluid along one face of the standard permeable dialysis membrane. The circuitous path takes the blood or fluid from the patient, through the machine and along the face of the membrane, and finally back to the subject. The other path provides for contact along the other face of the dialysis membrane by the dialysate or buffer solution (along with, typically, an appropriate amount of water) that will receive the toxins. Normally, the two paths run "countercurrent" to one another, such that the blood or fluid flows along the membrane in a first direction and the dialysate or buffer solution flows along the other face of the membrane in a direction generally opposite to the first direction. As contemplated herein, the dialysate or buffer solution may comprise a capturing and binding agent. Although the two solutions will be separated at all times by the dialysis membrane, all substances below the cut-off molecular weight of the dialysis membrane (e.g., the pore size of the permeable membrane) will be able to transfer back and forth. In a standard dialysis machine, the buffer solution may be continually replaced to increase the rate of extraction of toxins. If a capturing and binding agent having a particularly powerful binding strength is used however, it may not be necessary to replace the buffer solution. As previously described, the capturing arid binding agent may contain polypeptide which may be prepared to consist three or more amino acid sequences which are linked together through a linker with one or more capturing arms. Increasing the number of capturing arms of the linker increases the total length thereof. The total length of the capturing arms is directly proportional to the binding capacity of the capturing and binding agent such that a longer chain of capturing arms yields a greater capacity of the binding agent to capture and bind targeted Beta-Amyloids. In an embodiment above, the present invention advantageously provides an 8-arm assembly to achieve optimum binding capacity.

The standard dialysis membrane may be one of a generally synthetic based membrane or a traditional cellulose based dialysate membrane. Moreover, high flux hemodialysis membranes have new technologies that allow passage of larger size molecules through the membrane through larger pores supported by diffusion and convention. Alternatively, more recent nanotechnology versions are becoming available. Nanotechnology is being used in some of the most recent high-flux membranes to create a uniform pore size. The goal of high-flux membranes is to pass relatively large molecules such as beta-2 microglobulin (having an approximate molecular weight of 11,600 Daltons), but not to pass albumin (having an approximate molecular weight of 66,400 Daltons).

A single Beta-Amyloid strand is has an approximate molecular weight of 4,200 Daltons. However, we have identified that ADDLS, which is neurotoxic form Beta-Amyloids most prevalent in the body when there are plagues, are an aggregate of mostly 8 Beta-Amyloid peptides combined to have a resulting molecular weight of 33,600 Daltons. Thus, it is preferable to use a membrane which can allow passage to these larger molecules, such as membranes which may allow molecules with molecular weight as high as 45,000 Daltons to 50,000 Daltons to pass through them.

In one embodiment, the capturing and binding agent comprises the peptide sequence of KLVFF or a variant thereof, e.g., a retro or a reverse analog (see, e.g., Table 1 in Applicant's attached related application). This peptide may be linked to a poly(ethylene glycol) cross-linker/carrier gel to increase the total molecular weight of the linked carrier gel and said peptide sequence, thereby preventing its transport across the dialysis membrane. Such a capturing and binding agent and carrier can successfully bind to Beta-Amyloids, which may thus remove Beta-Amyloids from the blood or fluid passing through the dialysis machine and along the membrane. About 10mg to about 100mg of the carrier gel should be used per treatment and would suffice, though these quantities may vary, e.g., depending on various parameters of each particular patient. The capturing and binding agent, gel, and water combination may further comprise a standard dialysate since a high-flux hemodialysis treatment requires electrolytes and other elements to be put into the blood (the electrolytes and other elements may pass through the membrane and into the blood or fluid, as is commonly done in a typical dialysis treatment for pH balance and other considerations and as understood by one of ordinary skill in the art).

In a further embodiment, the capturing and binding agent may be configured such that the capturing and binding agent itself cannot pass through the pores of the selected membrane. However, a molecular size of approximately 33,600 Daltons is not necessarily required to achieve this since the synthetic gel does not fold like the Beta-Amyloid peptides (or ADDLS) found in the body. Thus, the capturing and binding agent may only need to have a molecular weight of approximately 15,000 Daltons to 20,000 Daltons to prevent passage through the pores of the membrane, since its hydrodynamic volume makes its apparent molecular weight large.

Such an enhanced capturing and binding agent may added to water or other conventional dialysis solution or buffer solution for use in the dialysis machine for dialysis treatment of a subject or patient suffering from AD or other pathological condition associated with abnormal in vivo levels of beta amyloid peptides. Alternatively, the carrier gel may still be used such that the enhanced capturing and binding agent is linked to the carrier gel. The combination of the capturing and binding agent and water (and optionally carrier gel) may further comprise a standard dialysate since a high-flux hemodialysis treatment requires electrolytes and other elements to be out into the blood (the electrolytes and other elements may pass through the membrane and into the blood or fluid, as is commonly done in a typical dialysis treatment for pH balance and other useful and related considerations).

In another embedment, the present invention may include a modified version of a typical dialysis machine for the treatment of AD, wherein a buffer solution is used with a dialysis machine, the buffer solution comprising a capturing and binding agent as described herein. The capturing and binding agent may be, for example, the KLVFF peptide sequence. Such a capturing and binding agent may increase the effectiveness of such a dialysis treatment to extract toxins found in patients with Alzheimer's Disease (AD) having Beta-Amyloid peptides. Such as system may be referred to as, for example, a Beta-Amyloid detoxification flush-style therapy.

In yet another embodiment, the present invention may include a system for treating a patient in need thereof, comprising, a dialysis machine which comprises a highly permeable membrane; and a dialysate comprising at least a carrier gel and a capturing and binding agent linked to the carrier gel. The dialysis machine may be any hemodialysis machine as that known in the art. The permeable membrane may be a synthetic membrane, including but not limited to membranes suitable for high flux dialysis. The carrier gel may be a poly(ethylene glycol) cross-linker/carrier gel, or a carrier protein as it is known in the art, or any other carrier molecule or device designed to retrieve the hydrodynamic volume or molecular weight of the trapping reagent. The capturing and binding agent may be a KLVFF peptide or a retro or a reverse analog or other variant thereof. Alternatively, the capturing and binding agent may have a larger molecular size than the KLVFF peptide. Further, such a capturing and binding agent may have a molecular size equivalent to a molecular weight of approximately 15,000 Daltons to 20,000 Daltons. The dialysate may further comprise water or other suitable buffer for dialysis. Such as a system may be used to treat a subject in need thereof, e.g., a patient with Alzheimer's Disease (AD) or any other Beta-Amyloid-related pathology wherein extraction of Beta-Amyloid peptides from a subject is desired.

As contemplated herein, a subject in need thereof includes a human suffering from a pathological condition associated with abnormal in vivo levels of Beta-Amyloid peptides (beta amyloids). In a particular embodiment, such pathological conditions are selected from the group consisting of Alzheimer's Disease, diabetes, Parkinson's Disease, Huntington's Disease, cataracts, muscular dystrophy, and Down's Syndrome.

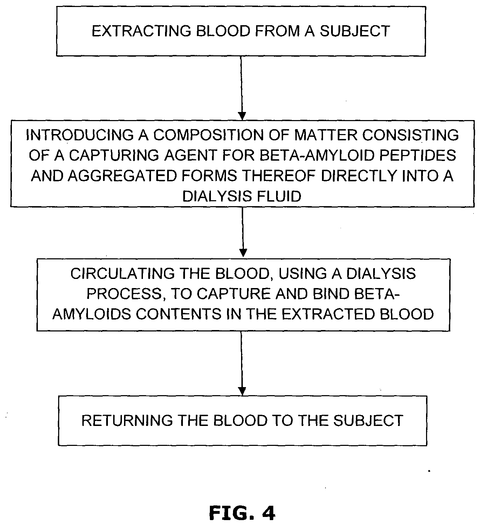

Referring now to FIG. 4, where is shown a flowchart illustrating a method of reducing Beta-Amyloid levels in the blood of a subject requiring such treatment by extracorporeal circulation. The method of reducing Beta-Amyloid levels in the blood of a subject requiring such treatment by extracorporeal circulation mainly comprises of four (4) primary steps. The first step is extracting blood from the subject at a pre-determined flowrate using a blood filtration device configured to separate plasma constituents from other cellular components of the blood. The blood filtration device includes a blood circuit side adapted to receive the extracted blood, a dialysate side adapted to receive a dialysis fluid, and a permeable membrane that separates the blood circuit side and the dialysate side. The second step, moving forward, is directed to the process of introducing an effective amount of a composition consisting mainly of a capturing and binding agent for Beta-Amyloid peptides directly into the dialysis fluid contained in the dialysate side. The capturing and binding agent has a binding capacity sufficient to attract and capture the neurotoxic Beta-Amyloid peptides from the plasma constituents circulating in the blood circuit side. In one preferred embodiment of the present invention, the permeable membrane is a semi-permeable type that has pore sizes that are substantially larger than the sizes of the Beta-Amyloid peptides, (i.e. 50,000 Daltons) thereby enabling the Beta-Amyloid peptides to pass through the semi-permeable membrane. Following this step is the third step, wherein the blood contained in the blood circuit side is being circulated therein. The circulation takes a substantially uniform direction or flow path. The blood circuit side, as it is known in art, may be situated in along the periphery of the side for the dialysis fluid or dialysate. This dialysate side is where the composition consisting of the capturing and binding agent is being introduced. The capturing and binding agent may then capture and bind the Beta-Amyloids, and as well as dimers and oligomers thereof, which are often present in the plasma composition of the blood. The circulation of the blood, through a regular hemodialysis process, provides the separation of the plasma. This enables the capturing and binding agent to capture and bind the Beta-Amyloids which pass through the semi-permeable membrane. Finally, the fourth step is returning the other cellular components and, subsequently, treated plasma constituents of the blood without Beta-Amyloids back to the subject. This step involves a path to the same membrane. The blood goes back into the body of the subject is now substantially free from Beta-Amyloids. The therapeutic effect of this method would then to prevent any pathological condition which is closely associated with abnormal level of Beta-Amyloids in vivo.

In one preferred embodiment, the present invention relates to a method of treating a subject suffering from a pathological condition associated with abnormal in vivo levels of Beta-Amyloid peptide comprising administering to said subject the dialysis detoxification procedure of the present invention to extract Beta-Amyloid peptides from the subject, and said extraction reduces levels of Beta-Amyloid peptides in the subject. In a particular embodiment, the pathological condition is selected from the group consisting of Alzheimer's Disease, Down's Syndrome, diabetes, Parkinson's Disease, Huntington's Disease, cataracts, and muscular dystrophy.

In another aspect, the invention relates to a method of reducing the likelihood of a subject developing a pathological condition associated with abnormal levels of Beta-Amyloid peptides comprising administering to said subject the dialysis detoxification procedure of the present invention for a time and in an amount sufficient to extract Beta-Amyloid peptides from the subject and said procedure results in reduced in vivo levels of Beta-Amyloid peptides in the subject.

Such a system and method, as both described above, may be used on the blood of a patient in need thereof, and further may be used as often as required, and may even be used repeatedly or continuously to remove such toxins from the blood. Moreover, the high efficiency of detoxification by the capturing and binding agent may also result in fewer frequency of treatments required.

In a further embodiment, the present invention may include a package for treatment of patients in need thereof, and the package may comprise a quantity of a carrier gel comprising a capturing and binding agent. The package may also comprise a dialysate solution or a dialysis fluid.

Under treatment procedures as described above, the Beta-Amyloids in the blood, along with its oligomer form (especially ADDLS), are bound to capturing and binding agent-gel enhanced dialysate as the Beta-Amyloids and ADDLS pass through the highly permeable membrane, thereby cleansing the blood of these toxins as the blood passes through the dialysis membrane.

Throughout this discussion, the term "dialysate" has been used synonymously with the terms "buffer solutions" and "dialysis fluid" to define the fluid which attracts and captures toxins from the blood or fluid as the blood or fluid pass through the dialysis machine.

### ADVANTAGES

The present invention provides the following advantages:
(1) It provides a binding action that is specific to Beta-Amyloids;
(2) It does not rely on the mechanical nature (e.g., filtration using a membrane) of Beta-Amyloid removal; instead, it simply utilizes a binding agent to capture Beta-amyloids from blood components;
(3) It provides a process by which highest binding potential is systematically created; and
(4) It provides a process which does not involve introducing of foreign substances in the body thereby eliminating potential immune system response which, in turn, may be translated into adverse risk events.

## Claims

1. A process for preparing a dialysis fluid formulation effective for extracorporeal treatment, through a blood filtration process, of a Beta-Amyloid associated pathological condition in a subject, said process comprises preparing a composition comprising KLVFF peptide, or a variant thereof, as the capturing and binding agent, and a carrier therefor, and mixing said composition with a dialysate solution, wherein the variant includes FFVLK peptide which is a reverse analog of KLVFF peptide.

2. A process according to claim 1, wherein the dialysis fluid consists of acid, water, and sodium bicarbonate, and wherein the dialysis fluid further consists of substances that are required to reestablish ionic equilibrium of the blood.

3. A process according to claim 1, wherein the subject has a pathological condition selected from a group consisting of: Alzheimer's Disease, diabetes, Parkinson's Disease, Huntington's Disease, cataracts, muscular dystrophy, and Down's Syndrome.

4. A process according to claim 1, wherein the capturing and binding agent, or one of its derivatives, is brought into dosage form together at least one solid, liquid, or semi-liquid carrier and/or auxiliary substance.

5. A process according to claim 1, wherein the capturing and binding agent is a tetramer peptide containing four copies of the monomer peptide in reverse sequence, as shown below:

NH2-lys--phe-phe-val-leu-lys-βala

NH2-lys--phe-phe-val-leu-lys-βala-lys

lys-cys-amide

NH2-lys--phe-phe-val-leu-lys-βala-lys

NH2-lys--phe-phe-val-leu-lys-βala

6. A process according to claim 5, wherein the tetramer capturing and binding agent is linked, through its cys side chain, to an 8- arm polyethylene glycol maleimide to form a molecule of the capturing and binding agent with 32 Beta-Amyloid capturing arms.

7. A process according to claim 1, wherein the blood filtration process consists of a blood filtration device configured to separate plasma constituents from other cellular components of the blood, wherein the blood filtration device includes a blood circuit side adapted to receive the extracted blood, a dialysate side adapted to receive the dialysis fluid formulation, and a permeable membrane that separates the blood circuit side and the dialysate side.

8. A process according to claim 7, **characterized in that** the capturing and binding agent has a binding capacity sufficient to attract and bind the neurotoxic Beta-Amyloid peptides from the plasma constituents circulating in the blood circuit side.

9. A process according to claim 7, **characterized in that** the permeable membrane is a semi-permeable membrane having pore sizes that are substantially larger than the sizes of the Beta- Amyloid peptides and an aggregated form thereof, whereby the Beta-Amyloid peptides and aggregated forms thereof are permitted to pass through the semi-permeable membrane.

10. A process according to claim 7, wherein blood filtration device is a hemodialysis machine.

11. A process according to claim 7, wherein the permeable membrane is **characterized by** a porous material which allows low molecular weight substances, including a wide array of toxins, to pass therethrough.

12. A process according to claim 9, wherein the semi-permeable membrane is any of the following: a synthetic based membrane and a cellulose based membrane.

## Patentansprüche

1. Ein Verfahren zur Herstellung einer Dialyseflüssigkeitsformulierung, die wirksam für die extrakorporale Behandlung eines mit Beta-Amyloid assoziierten pathologischen Zustandes in einem Subjekt durch ein Blut-Filtrationsverfahren ist, wobei jener Prozess die Herstellung einer Zusammensetzung, die das KLVFF Peptid oder einer Variante davon als Capturing- und Binde-Agens und einen Trägerstoff dafür umfasst, und das Mischen jener Zusammensetzung mit einer Dialyse-Lösung umfasst, worin die Variante das FFVLK Peptid einschließt, das ein umgekehrtes Analogon des KLVFF Peptids ist.

2. Ein Verfahren nach Anspruch 1, worin die Dialyseflüssigkeit aus Säure, Wasser und Natriumhydogencarbonat besteht und worin die Dialyseflüssigkeit ferner aus Substanzen besteht, die zur Wiederherstellung des Ionen-Gleichgewichts des Blutes nötig sind.

3. Ein Verfahren nach Anspruch 1, worin das Subjekt einen pathologischen Zustand hat, der ausgewählt ist aus der Gruppe bestehend aus: Alzheimer, Diabetes, Parkinson, Huntington, Katarakt, Muskeldystrophie und Down Syndrom.

4. Ein Verfahren nach Anspruch 1, worin das Capturing- und Binde-Agens, oder eines seiner Derivate, zusammen mit mindestens einem festen, flüssigen oder halb-flüssigen Trägerstoff und/oder einer Hilfssubstanz in eine Dosierungsform gebracht wird.

5. Ein Verfahren nach Anspruch 1, worin das Capturing- oder Binde-Agens ein Tetramer-Peptid ist, das vier Kopien des Monomer-Peptids in umgekehrter Sequenz enthält, wie unten gezeigt:

NH₂-lys--phe-phe-val-leu-lys-βala
NH₂-lys--phe-phe-val-leu-lys-βala-lys
lys-cys-amid
NH₂-lys--phe-phe-val-leu-lys-βala-lys
NH₂-lys--phe-phe-val-leu-lys-βala

6. Ein Verfahren nach Anspruch 5, worin das Tetramer Capturing- und Binde-Agens durch seine Cys Seitenkette an ein 8-armiges Polyethylenglykolmaleimid gebunden ist, um ein Capturing- und Binde-Agens mit 32 Beta-Amyloid Capturing-Armen zu bilden.

7. Ein Verfahren nach Anspruch 1, worin das Blut-Filtrationsverfahren aus einer Blut-Filtrationsvorrichtung besteht, die konfiguriert ist, um Plasma Bestandteile von anderen zellulären Komponenten des Blutes zu trennen, worin die Blut-Filtrationsvorrichtung eine Blutkreislaufseite einschließt, die adaptiert ist, um das extrahierte Blut zu empfangen, eine Dialysatseite, die adaptiert ist, die Dialyseflüssigkeitsformulierung zu empfangen und eine permeable Membran, die die Blutkreislaufseite und die Dialysatseite trennt.

8. Ein Verfahren nach Anspruch 7, darin charakterisiert, dass das Capturing- und Binde-Agens eine Bindekapazität hat, die ausreichend ist, um das neurotoxische Beta-Amyloid Peptid von den Plasma-Bestandteilen, die in der Blutkreislaufseite zirkulieren, anzuziehen und zu binden.

9. Ein Verfahren nach Anspruch 7, darin charakterisiert, dass die permeable Membran eine semi-permeable Membran ist, die eine Porengröße hat, die wesentlich größer ist als die Größe des Beta-Amyloid Peptids und einer aggregierten Form davon, wobei den Beta-Amyloid Peptiden und aggregierten Formen davon erlaubt ist, die semi-permeable Membran zu passieren.

10. Ein Verfahren nach Anspruch 7, worin die Blutfiltrations-Vorrichtung eine Haemodialyse-Maschine ist.

11. Ein Verfahren nach Anspruch 7, worin die permeable Membran durch ein poröses Material charakterisiert ist, das es niedermolekularen Substanzen, einschließlich eines breiten Spektrums an Toxinen, erlaubt, zu passieren.

12. Ein Verfahren nach Anspruch 9, worin die semi-permeable Membran eine der folgenden ist: eine synthetisch basierte Membran und eine Zellulose basierte Membran.

## Revendications

1. - Procédé de préparation d'une formulation de fluide de dialyse effective pour un traitement extracorporel, par un procédé de filtration du sang, d'un état pathologique associé au bêta-amyloïde chez un sujet, ledit procédé comprenant la préparation d'une composition comprenant le peptide KLVFF, ou un variant de celui-ci, comme agent de capture et de liaison, et un support associé, et le mélange de ladite composition avec une solution de dialysat, le variant comprenant le peptide FFVLK qui est un analogue inverse du peptide KLVFF.

2. - Procédé selon la revendication 1, dans lequel le fluide de dialyse consiste en acide, eau et bicarbonate de sodium, et dans lequel le fluide de dialyse consiste en outre en des substances qui sont requises pour rétablir un équilibre ionique du sang.

3. - Procédé selon la revendication 1, dans lequel le sujet a un état pathologique choisi dans un groupe consistant en : la maladie d'Alzheimer, le diabète, la maladie de Parkinson, la maladie de Huntington, la cataracte, la dystrophie musculaire et le syndrome de Down.

4. - Procédé selon la revendication 1, dans lequel l'agent de capture et de liaison, ou l'un de ses dérivés, est amené en une forme posologique conjointement avec au moins un support et/ou une substance auxiliaire solide, liquide ou semi-liquide.

5. - Procédé selon la revendication 1, dans lequel l'agent de capture et de liaison est un peptide tétramère contenant quatre copies du peptide monomère en séquence inverse, comme présenté ci-après :

NH2-lys--phe-phe-val-leu-lys-βala
NH2-lys--phe-phe-val-leu-lys-βala-lys
lys-cys-amide
NH2-lys--phe-phe-val-leu-lys-βala-lys
NH2-lys--phe-phe-val-leu-lys-βala

6. - Procédé selon la revendication 5, dans lequel l'agent de capture et de liaison tétramère est lié, par sa chaîne latérale cys, à un polyéthylène glycol maléimide à 8 bras pour former une molécule de l'agent de capture et de liaison avec 32 bras de capture de bêta-amyloïde.

7. - Procédé selon la revendication 1, dans lequel le procédé de filtration du sang consiste en un dispositif de filtration de sang configuré pour séparer des constituants du plasma à partir des autres composants cellulaires du sang, dans lequel le dispositif de filtration du sang comprend un côté circuit de sang apte à recevoir le sang extrait, un côté dialysat apte à recevoir la formulation de fluide de dialyse, et une membrane perméable qui sépare le côté circuit de sang et le côté dialysat.

8. - Procédé selon la revendication 7, **caractérisé par le fait que** l'agent de capture et de liaison a une capacité de liaison suffisante pour attirer et lier les peptides bêta-amyloïdes neurotoxiques provenant des constituants du plasma circulant dans le côté circuit de sang.

9. - Procédé selon la revendication 7, **caractérisé par le fait que** la membrane perméable est une membrane semi-perméable ayant des dimensions de pore qui sont sensiblement plus grandes que les dimensions des peptides bêta-amyloïdes et une forme agrégée de ceux-ci, ce par quoi les peptides bêta-amyloïdes et les formes agrégées de ceux-ci sont autorisés à passer à travers la membrane semi-perméable.

10. - Procédé selon la revendication 7, dans lequel le dispositif de filtration du sang est une machine d'hémodialyse.

11. - Procédé selon la revendication 7, dans lequel la membrane perméable est **caractérisée par** une matière poreuse qui permet à des substances de faible masse moléculaire, comprenant un large ensemble de toxines, de passer à travers elle.

12. - Procédé selon la revendication 9, dans lequel la membrane semi-perméable est l'une quelconque des suivantes : une membrane à base synthétique et une membrane à base de cellulose.
